# EUROPÄISCHE PATENTSCHRIFT

(11) **EP 0 599 891 B1**
(45) Veröffentlichungstag und Bekanntmachung des Hinweises auf die Patenterteilung: **08.11.1995**
(21) Anmeldenummer: 92916796.3
(22) Anmeldetag: 08.08.1992
(51) Int. Cl.: C12P 35/06

(54) **VERFAHREN ZUR FERMENTATIVEN HERSTELLUNG VON CEPHALOSPORIN C MIT ACREMONIUM CHRYSOGENUM**
METHOD FOR THE FERMENTATIVE PRODUCTION OF CEPHALOSPORIN C USING ACREMONIUM CHRYSOGENUM
PROCEDE DE FABRICATION, PAR FERMENTATION, DE CEPHALOSPORINE C AU MOYEN D'ACREMONIUM CHRYSOGENUM

(30) Priorität: 21.08.1991 DE 4127648
(43) Veröffentlichungstag der Anmeldung: 08.06.1994
(73) Patentinhaber: HOECHST AKTIENGESELLSCHAFT, 65926 Frankfurt am Main (DE)
(72) Erfinder: BAYER, Thomas, D-6232 Bad Soden am Taunus (DE); SCHRAMM, Wilhelm, D-6233 Kelkheim (DE); RATHSCHECK, Wolfgang, D-6232 Bad Soden am Taunus (DE)
(86) Internationale Anmeldenummer: EP9201811
(87) Internationale Veröffentlichungsnummer: WO9304188

(56) Entgegenhaltungen:
- Contributed Paper, chapter 32, pages 455-470, M. Kalyanpur et al:
- J. Chem. Tech. Biotechnol., Vol. 42, 1988 Tim A.J. Harris et al.: see page 19-page 30

## Beschreibung

Die Aufarbeitung von Antibiotika enthaltenden Kulturbrühen erfolgt in der Regel nach dem Erreichen des maximalen Gehalts. Meistens erfolgt als erster Schritt eine Abtrennung von Zellen und Feststoffen durch Filtration oder Zentrifugation. Danach kann eine Wertstoffanreicherung durch Extraktion oder Adsorption erfolgen. Das gereinigte Produkt wird dann in der Regel kristallisiert und kann zu semisynthetischen Antibiotika weiterverarbeitet werden.

Bei dieser Schritt-für-Schritt Methode macht sich die Instabilität vieler Antibiotikamoleküle, wie z.B. Cephalosporin C (CPC) nachteilig bemerkbar. Schon während der Fermentation findet ein Abbau des CPC's statt. Dieser kann sowohl rein chemisch, Angriff von Wasser auf den β-Lactamring, als auch enzymatisch, z.B. durch Esterasen, stattfinden (Konecny et al., 1973, J. of Antib., 26, 3, 135-141). Ferner treten bei der Fermentation eine Reihe von Nebenprodukten, wie Deacetoxycephalosporin C (DOCPC) und Deacetylcephalosporin C (DCPC) auf, die bei der Reinigung von CPC abgetrennt werden müssen. Damit sind erhebliche Ausbeuteverluste verbunden. Cross-flow-Filtrationssysteme mit Polymer- oder Keramikmembranen wurden für die Aufarbeitung von Antibiotikabrühen bereits verwendet (Harris et al., J. Chem. Techn. Biotechnol., 1988, 42, 19-30). Es wurde bisher nicht gezeigt, daß bei diesen Verfahren der Zerfall von Cephalosporin C vermindert werden kann. Es ist bekannt, Cephalosporin C nach der Fermentation mit Cross-flow-Filtration zu isolieren (M. Kalyanpur et al., Contributed Paper, Kapitel 32, Seiten 455-470).

Aufgabe der vorliegenden Erfindung ist es, ein Verfahren zu finden, mit dem der Abbau von Cephalosporin C und die Nebenproduktbildung vermindert, sowie die Ausbeute bezogen auf die eingesetzte Substratmenge erhöht werden können.

Überraschenderweise wurde nun gefunden, daß durch Verwendung eines Cross-flow Filtrationsmoduls während der Fermentation von Acremonium chrysogenum die Ausbeute an CPC erhöht, die Bildung von DCPC vermindert und die Produktionszeit verlängert werden konnte. Ferner wird durch die Filtration und die geringe DCPC-Bildung die Aufarbeitung von Cephalosporin C wesentlich vereinfacht.

Die Erfindung betrifft daher ein Verfahren zur Herstellung von CPC, das dadurch gekennzeichnet ist, daß die Fermentationslösung während der Fermentation über ein Cross-flow Filtrationssystem filtriert wird und die abgenommene Filtratmenge im Fermenter ersetzt werden kann.

Acremonium chrysogenum (Cephalosporium acremonium), sowie Mutanten und Selektanten, solange sie CPC-Derivate herstellen, eignen sich zum Einsatz im erfindungsgemäßen Verfahren.
Die Nährlösung enthält Kohlenstoffquellen wie Saccharose, Maisstärke, Dextrose oder Molasse und Stickstoffquellen wie Sojabohnenmehl, Erdnußmehl, Malzextrakt oder Ammoniumacetat.
Das Nährmedium enthält auch anorganische Salze wie Natriumhydrogenphosphat, Natriumchlorid, Calciumchlorid, Calciumsulfat, Calciumcarbonat, Magnesiumsulfat oder Kaliumhydrogenphosphat. Ferner kann dem Nährmedium auch Fett wie Ölsäuremethylester oder Sojaöl zugesetzt werden. Daneben werden auch Spurenelemente wie Eisen-, Mangan-, Kupfer-, Zink-, Kobalt- oder andere Metallsalze zugegeben.

Die Kultivierung von Acremonium chrysogenum (Chephalosporium acremonium), bevorzugt DSM 6473, erfolgt bei Temperaturen zwischen 20°C und 30°C, bevorzugt bei 25°C und bei pH-Werten zwischen 5 und 8, bevorzugt bei pH 7. Die Kultivierung erfolgt aerob zunächst im Schüttelkolben und danach im Fermenter unter Rühren und Belüftung mit Luft oder reinem Sauerstoff. Die Kultivierung der Mikroorganismen in den Fermentern erfolgt über einen Zeitraum von 120 bis 240 Stunden, bevorzugt zwischen 130 und 170 Stunden.

Als Cross-flow Fitrationssystem können Platten-, Rohr-, Kapillarrohr-, Wickel- oder Hohlfasermembranmodule aus Polymeren, Kohlenstoff oder Keramik mit Trenngrenzen vom Ultrafiltrations- bis zum Sterilfiltrationsbereich eingesetzt werden. Bevorzugt werden Filtrationsmodule mit einer Porengröße von 0,2 µm oder 4 nm eingesetzt. Als Membranmaterialien können Polysulfone, Polyamide, Celluloseacetat, Aluminiumoxid oder Zirkonoxid eingesetzt werden.
Die Filtration kann kontinuierlich oder diskontinuierlich erfolgen. Sie beginnt etwa 2 - 3 Tage nach dem Animpfen des Fermenters und kann bis zum Ende der Fermentation fortgeführt werden. Die Überströmgeschwindigkeit der Fermenterlösung über die Filtrationsfläche beträgt zwischen 0,5 und 20 m/s, bevorzugt 1 bis 10 m/s.
Das Filtrat, das während der Fermentation aus dem Fermenter abgezogen wird, kann durch eine entsprechende Menge an Flüssigkeit ersetzt werden oder nach Abtrennung des Wertproduktes z.B. durch Absorbtion, kann das Filtrat in den Fermenter zurückgeführt werden. Dazu kann Wasser, angereichert mit entsprechenden Salzen oder anderen Nährmediumsbestandteilen, zugepumpt werden.

Das nicht durch die Membran permeierte Flüssigkeitsvolumen wird wieder in den Fermenter zurückgeführt.
Der Fermenter und das Cross-flow Filtrationssystem werden mit entsprechenden Rohren oder Schläuchen verbunden und vor der Fermentation sterilisiert. Für einen 100l-Fermenter werden etwa 0,2 m² Filtrationsfläche benötigt. Es können auch größere und kleinere Filtrationsflächen verwendet werden.

### Beispiel 1:

### Fermentation von Acremonium chrysogenum DSM 6473

Die Fermentation erfolgte in folgender Nährlösung:

| Vorkulturmedium | g/l |
|---|---|
| Cornsteep | 11,75 |
| Ammonium acetat | 4,5 |
| Saccharose | 20,0 |
| CaSO₄·2H₂O | 0,5 |
| MgSO₄·7H₂O | 0,5 |
| pH 7,0 (eingestellt mit 15 w% NaOH) | |

| Fermentationsmedium | g/l |
|---|---|
| fettfreies Erdnußmehl | 100,0 |
| Ammonium Acetat | 6,0 |
| Glucose Monohydrat | 5,0 |
| Methyloleat | 5,0 |
| D,L-Methionin | 3,0 |
| CaSO₄·2H₂O | 5,0 |
| MgSO₄·7H₂O | 5,0 |
| CaCO₃ | 5,0 |
| Antischaummittel | 0,5 |

| Fed-batch Lösung: | |
|---|---|
| Glucose Monohydrat | 500,0 |
| D,L-Methionin | 24,75 |

Schrägagarkulturen werden zur Beimpfung von 100 ml Vorkulturmedium verwendet (500 ml Schüttelkolben mit 4 Schikanen). Die Kolben werden 48 Stunden mit 150 Umdrehungen pro Minute (Upm) bei 25 bis 28°C inkubiert. Diese Kulturen werden zum Beimpfen einer weiteren Vorkultur verwendet (1000 ml Medium, 5000 ml Kolben, 25 bis 28°C, 120 Upm, 58 bis 60 Stunden). Mit der zweiten Vorkultur werden 60 l Fermentationsmedium in einem Rührfermenter beimpft. Die Fermentation erfolgt bei 25°C. Die Begasung wird so gesteuert, daß der pO₂ über 20 % in der Fermentationsnährlösung liegt.

Nach 74 Stunden Fermentation beginnt die Filtration mit einem Cross-flow-Keramikmodul (Fa. Membraflow, α-Al₂O₃) mit 0,2 m² Filterfläche und 0,2 µm Porengroße. Es wurden folgende Prozeßparameter eingehalten:
- Überströmgeschwindigkeit:: 2 m/s
- Umpumpgeschwindigkeit:: 1500 l/h
- Filtrationsleistung:: 2 l/h
- Fitrationszeit:: 68 Std.

Die nachfolgende Tabelle zeigt die Versuchsergebnisse nach 142 Stunden einer Fermentation ohne (A) und mit Cross-flow Filtration (B):

**Tabelle 1**

| | CPC [%] | DCPC/CPC [%] | Produktivität CPC | | Produktivität DCPC nach 6 Tagen [%] |
|---|---|---|---|---|---|
| | | | max. [%] | nach 6 Tagen [%] | |
| A | 100 | 100 | 100 | 100 | 100 |
| B | 140 | 44 | 200 | 400 | 53 |

### Beispiel 2

Die Fermentation erfolgt wie in Beispiel 1. Tabelle 2 zeigt die Ergebnisse nach 167 Stunden einer Fermentation mit Cross-flow Filtration (B) im Vergleich zu einer Parallel-Fermentation ohne Filtration die nach 142 Stunden abgebrochen wurde:

**Tabelle 2**

| | CPC (%) | DCPC/CPC (%) |
|---|---|---|
| A (142 Stunden) | 100 | 100 |
| B (167 Stunden) | 129 | 69 |

## Patentansprüche

1. Verfahren zur fermentativen Herstellung von Cephalosporin C mit Acremonium chrysogenum, dadurch gekennzeichnet, daß die Fermentationslösung während der Fermentation über ein Cross-flow Filtrationssystem filtriert wird und die Filtratmenge im Fermenter ersetzt werden kann.

2. Verfahren nach Anspruch 1, dadurch gekennzeichnet, daß ein Polymer- oder Keramikfilter als Cross-flow Filtrationssystem verwendet wird.

3. Verfahren nach Anspruch 1 oder 2, dadurch gekennzeichnet, daß das Filter eine Porengröße zwischen 4 und 200 nm hat.

4. Verfahren nach einem oder mehreren der Ansprüche 1 bis 3, dadurch gekennzeichnet, daß die Überströmgeschwindigkeit über die Filterfläche 1 bis 10 m/s beträgt.

5. Verfahren nach einem oder mehreren der Ansprüche 1 bis 4, dadurch gekennzeichnet, daß die Filtratlösung durch Wasser im Fermenter ersetzt wird.

6. Verfahren nach Anspruch 5, dadurch gekennzeichnet, daß die Filtratlösung durch eine Nährlösung ersetzt wird.

7. Verfahren nach Anspruch 6, dadurch gekennzeichnet, daß die Filtratlösung nach Entfernung des Wertproduktes in den Fermenter zurückgeführt wird.

## Claims

1. A process for the fermentative production of cephalosporin C using Acremonium chrysogenum, which comprises filtering the fermentation solution during the fermentation through a cross-flow filtration system and the possibility of replacing the amount of filtrate in the fermenter.

2. The process as claimed in claim 1, wherein a polymer filter or ceramic filter is used as the cross-flow filtration system.

3. The process as claimed in claim 1 or 2, wherein the filter has a pore size of between 4 and 200 nm.

4. The process as claimed in one or more of claims 1 to 3, wherein the flow rate over the filter surface is 1 to 10 m/s.

5. The process as claimed in one or more of claims 1 to 4, wherein the filtrate solution in the fermenter is replaced by water.

6. The process as claimed in claim 5, wherein the filtrate solution is replaced by a nutrient solution.

7. The process as claimed in claim 6, wherein the filtrate solution is recycled to the fermenter after the valuable product has been removed.

## Revendications

1. Procédé pour la production par fermentation de céphalosporine C au moyen d'*Acremonium chrysogenum*, caractérisé en ce que la solution de fermentation est filtrée, pendant la fermentation, au moyen d'un système de filtration à écoulement transversal, et la quantité de filtrat peut être remplacée dans le fermentateur.

2. Procédé selon la revendication 1, caractérisé en ce que, en tant que système de filtration à écoulement transversal, on utilise un filtre céramique ou en polymère.

3. Procédé selon la revendication 1 ou 2, caractérisé en ce que le filtre a une taille de pores comprise entre 4 et 200 nm.

4. Procédé selon une ou plusieurs des revendications 1 à 3, caractérisé en ce que la vitesse de passage sur la surface du filtre est de 1 à 10 m/s.

5. Procédé selon une ou plusieurs des revendications 1 à 4, caractérisé en ce que la solution de filtrat est remplacée par de l'eau dans le fermenteur.

6. Procédé selon la revendication 5, caractérisé en ce que la solution de filtrat est remplacée par une solution nutritive.

7. Procédé selon la revendication 6, caractérisé en ce que la solution de fermentation est renvoyée dans le fermenteur, après extraction du produit recherché.
